# EUROPEAN PATENT APPLICATION

(11) **EP 4 583 118 A1**
(43) Date of publication of application: **09.07.2025**
(21) Application number: 24150173.3
(22) Date of filing: 03.01.2024
(51) Int. Cl.: G16H 40/20, G06Q 10/0633, G06Q 10/06

(54) **CLINICAL PROCEDURE EVENT PREDICTION**

(71) Applicant: Koninklijke Philips N.V., 5656 AG Eindhoven (NL)
(72) Inventor: BRATKE, Grischa, 5656 AG Eindhoven (NL); SONNABEND, Kristina, 5656 AG Eindhoven (NL); AMTHOR, Thomas Erik, 5656 AG Eindhoven (NL)
(74) Representative: Philips Intellectual Property & Standards

(57) **Abstract**

Aspects and embodiments disclosed relate to a method and system for clinical workflow management, and in particular to a method and system for event prediction in a clinical procedure. According to various, but not necessarily all, example embodiments described, there is provided a method for clinical workflow management. The method comprises: obtaining clinical workflow information comprising a plurality of events associated with a clinical procedure; obtaining case information associated with a group of the plurality of events which together form an occurrence of the clinical procedure, the case information comprising information relating to occurrence of at least some of the events in the group; evaluating, based on the clinical workflow information and case information: an indication of one or more schedule of the plurality of events associated with the clinical procedure; and a time difference between the events of the one or more schedule of events associated with the clinical procedure; and providing, based upon the evaluation, information relating to occurrence of an event of interest if provided with information relating to occurrence of another event associated with the clinical procedure. Aspects and embodiments recognise that it would be beneficial if a mechanism were provided to allow a workflow management or workflow monitoring application to estimate when which tasks or events in a workflow or process are likely to occur, and consequently to mitigate or eliminate delays or extensive communication needs relating to those tasks or events.

## Description

### FIELD OF THE INVENTION

The present invention relates to a method and system for clinical workflow management, and in particular to a method and system for event prediction in a clinical procedure.

### BACKGROUND OF THE INVENTION

Workflow management and real-time workflow monitoring applications are gaining importance in hospitals and other health care facilities. Tracking and monitoring processes which occur in a clinical or healthcare domain in real time support approaches which allow more appropriate allocation of resource in such environments.

Whilst real-time process tracking and monitoring can offer a wealth of information and control to users including, for example, healthcare professionals, such a wealth of information may result in information overload, with a result that meaningful or useful information can be lost or obfuscated.

Improvements relating to mechanisms for processing tracking and monitoring information could be of benefit in workflow management and monitoring applications.

### SUMMARY OF THE INVENTION

The scope of protection sought for various example embodiments of the invention is set out in the independent claims. The example embodiments and features, if any, described in this specification that do not fall under the scope of the independent claims are to be interpreted as examples useful for understanding various embodiments of the invention.

According to various, but not necessarily all, example embodiments there is provided a method for clinical workflow management, the method comprising: obtaining clinical workflow information comprising a plurality of events associated with a clinical procedure; obtaining case information associated with a group of the plurality of events which together form an occurrence of the clinical procedure, the case information comprising information relating to occurrence of at least some of the events in the group; evaluating, based on the clinical workflow information and case information: an indication of one or more schedule of the plurality of events associated with the clinical procedure; and a time difference between the events of the one or more schedule of events associated with the clinical procedure; and providing, based upon the evaluation, information relating to occurrence of an event of interest if provided with information relating to occurrence of another event associated with the clinical procedure.

It is recognised that a quantity of available information relating to a workflow may be significant and that a user may not have sufficient time, skill and/or experience to observe a dashboard or process tracking application and extract or observe pertinent consequences which can flow from provision of such information.

It is further recognised that a user of a workflow management or workflow monitoring application may only be interested in specific events which form part of a workflow or process.

It is yet further recognised that displaying unfiltered information, including real-time process information, can be problematic in terms of data privacy regulations.

It would be beneficial if a mechanism were provided to allow a workflow management or workflow monitoring application to estimate when which tasks or events in a workflow or process are likely to occur, and consequently to mitigate or eliminate delays or extensive communication needs relating to those tasks or events.

The time difference between events may comprise a projected or predicted elapsed time between the events of the one or more schedule of events associated with the clinical procedure.

The information relating to occurrence of at least some of the events in the group may comprise a determination of a time stamp.

Obtaining case information may comprise obtaining case information relating to a plurality of occurrences of the clinical procedure.

In some embodiments, the method comprises: providing a reasoning engine and configuring the reasoning engine to: obtain the clinical workflow information and case information; and create a model of the clinical procedure by evaluating the indication of one or more schedule and time difference between events.

In some embodiments, the model of the clinical procedure is created using historic clinical workflow information and case information.

In some embodiments, the method comprises: evaluating event scheduling and/or context information when evaluating the time difference between events in the schedule of events.

In some embodiments, the information relating to occurrence of another event associated with the clinical procedure comprises: real-time or near real-time information relating to an ongoing occurrence of the clinical procedure.

In some embodiments, the method comprises monitoring a clinical procedure based upon the provided information relating to occurrence of an event of interest.

In some embodiments, the method comprises: allocating clinical resource based upon the provided information relating to occurrence of an event of interest.

In some embodiments, the method comprises: estimating, based upon the evaluation, a time of occurrence of the event of interest.

In some embodiments, providing information relating to occurrence of the event of interest comprises a determination of whether the event of interest will occur.

In some embodiments, providing information relating to occurrence of the event of interest comprises a determination of when the event of interest will occur.

In some embodiments, providing information relating to occurrence of the event of interest comprises providing a determination of confidence or probability associated with the provided information.

In some embodiments, the method comprises: receiving a determination of one or more event of interest in an ongoing occurrence of the clinical procedure; and providing the information relating to occurrence of the one or more event of interest comprises providing the information in response to provision of information relating to occurrence of another event in the ongoing occurrence of the clinical procedure.

In some embodiments, the method comprises: notifying a user of the information relating to occurrence of the event of interest.

In some embodiments, the method comprises: generating an alert based upon the information relating to occurrence of the event of interest.

In some embodiments, generating an alert comprises: notifying a user of a time of occurrence of the event of interest.

In some embodiments, notifying a user comprises: notifying the user a predetermined period before a time of occurrence of the event of interest.

In some embodiments, the notification or alert comprises a visual, audio or haptic notification or alert.

In some embodiments, notifying a user comprises transmitting a notification to a remote user.

In some embodiments, the method comprises: receiving input from a user in response to notification of information relating to occurrence of the event of interest, the input from the user comprising information relating to one or more event of the plurality of events associated with the clinical procedure.

In some embodiments, the method comprises: obtaining key event information associated with a plurality of occurrences of the clinical procedure, the key event information comprising a determination of one or more event of interest to a user; and
providing a user with a suggestion of one or more event of interest in a clinical procedure based upon the obtained key event information.

In some embodiments, providing a reasoning engine and configuring the reasoning engine to: obtain the key event information and clinical workflow information; and generate a model of probable events of interest to a user of the plurality of events associated with the clinical procedure.

In some embodiments, the model of the clinical procedure is generated using historic clinical workflow information and key event information.

According to various, but not necessarily all, example embodiments there is provided a non-transitory computer readable medium comprising program instructions stored thereon for performing at least the following: obtaining clinical workflow information comprising a plurality of events associated with a clinical procedure; obtaining case information associated with a group of the plurality of events which together form an occurrence of the clinical procedure, the case information comprising information relating to occurrence of at least some of the events in the group; evaluating, based on the clinical workflow information and case information: a determination of one or more schedule of the plurality of events associated with the clinical procedure; and
a time difference between the events of the one or more schedule of events associated with the clinical procedure; and providing, based upon the evaluation, information relating to occurrence of an event of interest if provided with information relating to occurrence of another event associated with the clinical procedure.

The instructions may be for performing the optional features set out in relation to the method mentioned above.

According to various, but not necessarily all, example embodiments there is provided a computer program product operable, when executed on a computer, to perform steps of obtaining clinical workflow information comprising a plurality of events associated with a clinical procedure; obtaining case information associated with a group of the plurality of events which together form an occurrence of the clinical procedure, the case information comprising information relating to occurrence of at least some of the events in the group; evaluating, based on the clinical workflow information and case information: a determination of one or more schedule of the plurality of events associated with the clinical procedure; and a time difference between the events of the one or more schedule of events associated with the clinical procedure; and providing, based upon the evaluation, information relating to occurrence of an event of interest if provided with information relating to occurrence of another event associated with the clinical procedure.

The computer program product may be operable, when executed on a computer, to perform the optional features set out in relation to the method mentioned above.

According to various, but not necessarily all, example embodiments there is provided an apparatus comprising: at least one processor; and at least one memory storing instructions that when executed by the at least one processor cause the apparatus at least to perform: obtaining clinical workflow information comprising a plurality of events associated with a clinical procedure; obtaining case information associated with a group of the plurality of events which together form an occurrence of the clinical procedure, the case information comprising information relating to occurrence of at least some of the events in the group; evaluating, based on the clinical workflow information and case information: a determination of one or more schedule of the plurality of events associated with the clinical procedure; and a time difference between the events of the one or more schedule of events associated with the clinical procedure; and providing, based upon the evaluation, information relating to occurrence of an event of interest if provided with information relating to occurrence of another event associated with the clinical procedure.

The time difference between events may comprise a projected or predicted elapsed time between the events of the one or more schedule of events associated with the clinical procedure.

The information relating to occurrence of at least some of the events in the group may comprise a determination of a time stamp.

Obtaining case information may comprise obtaining case information relating to a plurality of occurrences of the clinical procedure.

In some embodiments, the apparatus may comprise: a reasoning engine configured to: obtain the clinical workflow information and case information; and create a model of the clinical procedure by evaluating the determination of one or more schedule and time difference between events.

In some embodiments, the model of the clinical procedure is created using historic clinical workflow information and case information.

In some embodiments, the apparatus is configured to evaluate event scheduling and/or context information when evaluating the time difference between events in the schedule of events.

In some embodiments, the information relating to occurrence of another event associated with the clinical procedure comprises: real-time or near real-time information relating to an ongoing occurrence of the clinical procedure.

In some embodiments, the apparatus is configured to monitor a clinical procedure based upon the provided information relating to occurrence of an event of interest.

In some embodiments, the apparatus is configured to allocate clinical resource based upon the provided information relating to occurrence of an event of interest.

In some embodiments, the apparatus is configured to estimate, based upon the evaluation, a time of occurrence of the event of interest.

In some embodiments, providing information relating to occurrence of the event of interest comprises a determination of whether the event of interest will occur.

In some embodiments, providing information relating to occurrence of the event of interest comprises a determination of when the event of interest will occur.

In some embodiments, providing information relating to occurrence of the event of interest comprises providing a determination of confidence or probability associated with the provided information.

In some embodiments, the apparatus is configured to receive a determination of one or more event of interest in an ongoing occurrence of the clinical procedure; and provide the information relating to occurrence of the one or more event of interest by providing the information in response to provision of information relating to occurrence of another event in the ongoing occurrence of the clinical procedure.

In some embodiments, the apparatus is configured to notify a user of the information relating to occurrence of the event of interest.

In some embodiments, the apparatus is configured to generate an alert based upon the information relating to occurrence of the event of interest.

In some embodiments, generating an alert comprises: notifying a user of a time of occurrence of the event of interest.

In some embodiments, notifying a user comprises: notifying the user a predetermined period before a time of occurrence of the event of interest.

In some embodiments, the notification or alert comprises a visual, audio or haptic notification or alert.

In some embodiments, notifying a user comprises transmitting a notification to a remote user.

In some embodiments, the apparatus is configured to receive input from a user in response to notification of information relating to occurrence of the event of interest, the input from the user comprising information relating to one or more event of the plurality of events associated with the clinical procedure.

In some embodiments, the apparatus is configured to obtain key event information associated with a plurality of occurrences of the clinical procedure, the key event information comprising a determination of one or more event of interest to a user; and
provide a user with a suggestion of one or more event of interest in a clinical procedure based upon the obtained key event information.

In some embodiments, the apparatus comprises a reasoning engine configured to: obtain the key event information and clinical workflow information; and generate a model of probable events of interest to a user of the plurality of events associated with the clinical procedure.

In some embodiments, the model of the clinical procedure is generated using historic clinical workflow information and key event information.

According to various, but not necessarily all, example embodiments there is provided an apparatus comprising: means configured to obtain clinical workflow information comprising a plurality of events associated with a clinical procedure; means configured to obtain case information associated with a group of the plurality of events which together form an occurrence of the clinical procedure, the case information comprising information relating to occurrence of at least some of the events in the group; means configured to evaluate, based on the clinical workflow information and case information: a determination of one or more schedule of the plurality of events associated with the clinical procedure; and a time difference between the events of the one or more schedule of events associated with the clinical procedure; and means configured to provide, based upon the evaluation, information relating to occurrence of an event of interest if provided with information relating to occurrence of another event associated with the clinical procedure.

The means may perform the optional features set out in relation to the apparatus mentioned above.

According to various, but not necessarily all, example embodiments there is provided an apparatus comprising: circuitry configured to obtain clinical workflow information comprising a plurality of events associated with a clinical procedure; circuitry configured to obtain case information associated with a group of the plurality of events which together form an occurrence of the clinical procedure, the case information comprising information relating to occurrence of at least some of the events in the group; circuitry configured to evaluate, based on the clinical workflow information and case information: a determination of one or more schedule of the plurality of events associated with the clinical procedure; and a time difference between the events of the one or more schedule of events associated with the clinical procedure; and circuitry configured to provide, based upon the evaluation, information relating to occurrence of an event of interest if provided with information relating to occurrence of another event associated with the clinical procedure.

The circuitry may be configured perform the optional features set out in relation to the apparatus mentioned above.

Further particular and preferred aspects are set out in the accompanying independent and dependent claims. Features of the dependent claims may be combined with features of the independent claims as appropriate, and in combinations other than those explicitly set out in the claims.

Where an apparatus feature is described as being operable to provide a function, it will be appreciated that this includes an apparatus feature which provides that function or which is adapted or configured to provide that function.

Mechanisms described recognise it would be helpful to provide a user with a mechanism to provide a reduced quantity of information, for example, information pertinent to a relevant event or task, based upon the available wealth of information which may be received by a system.

It may be helpful to provide a dedicated notification or alert system to provide a user with, for example, a reduced amount of all information available in relation to a clinical procedure. It would be helpful, for example, to notify an individual or entity in advance of occurrence of an event of interest.

It would be helpful to allow a user to select or "tag" an event, task or activity of interest which may occur as part of a clinical procedure and to provide information related to, or associated with, that event to the user. The selected or tagged event, task or activity, may be one which is of special interest in the clinical procedure.

It would be helpful to support a user by suggesting one or more event(s) of interest in relation to a clinical procedure.

It would be helpful to automatically notify medical professionals about events of interest and required actions related to their work without having to actively monitor progress of clinical cases. Such automatic notification may allow medical professionals to focus on their clinical activities whilst making sure that they are alerted ahead of time about pre-defined events of interest, thereby maximizing the time for concentrated work and mitigating disruption to departmental workflow.

These and other aspects of the invention will be apparent from and elucidated with reference to the embodiment(s) described hereinafter.

### BRIEF DESCRIPTION OF THE DRAWINGS

Some example embodiments will now be described with reference to the accompanying drawings in which:
FIG. 1 illustrates schematically components of one embodiment of a system configurable to operate in accordance with the subject matter described herein;
FIG. 2 is a representation of a possible user interface according to one implementation which may be displayed to a user in one embodiment of a system;
FIG. 3 illustrates schematically one possible mechanism for providing an event tagging prediction in one embodiment of a system;
FIG. 4 is a schematic representation of an apparatus according to one implementation; and
FIG. 5 is a schematic representation of a method according to one implementation.

### DETAILED DESCRIPTION OF EMBODIMENTS

Before discussing the example embodiments in any more detail, first an overview will be provided.

As described above, in a clinical or healthcare environment, workflow monitoring systems can be made available. In such systems, information relating to events or tasks which occur as part of a clinical process or procedure may be received. That information may include real-time or near real-time information. Analysis and/or processing of received information can support tracking and/or monitoring of a clinical process or procedure.

By way of example, a radiologist may be interested in receiving real-time information about a specific activity forming part of a clinical process, for example, a specific patient's imaging procedure. That radiologist may not, however, be able to continuously monitor the real-time status of the imaging procedure.

It would be helpful to provide a user with a mechanism to provide a reduced quantity of information, for example, information pertinent to a relevant event or task, based upon the available wealth of information which may be received by a system.

Approaches described may provide a method for clinical workflow management. The method may comprise steps including:
(i) Obtaining clinical workflow information comprising a plurality of events associated with a clinical procedure. The events associated with a clinical procedure may include sets of events which together define a task which may occur as part of a clinical procedure. A plurality of tasks may be associated with a clinical procedure. Not all events or tasks associated with a clinical procedure may occur in an instance or occurrence of a given clinical procedure.
(ii) Obtaining case information associated with a group of the plurality of events which together form an occurrence of the clinical procedure, the case information comprising information relating to occurrence of at least some of the events in the group. A group of events may together form an occurrence of the clinical procedure. Not all events of an available plurality of events may occur in a given occurrence of the clinical procedure. The information relating to occurrence of at least some of the events in the group comprises a determination of a time of occurrence of one or more event. The determination of a time of occurrence may comprise a time stamp, an initiation time, a completion time or similar. Obtaining case information may comprise obtaining case information relating to a plurality of occurrences or instances of the clinical procedure.
(iii) Evaluating, based on the clinical workflow information and case information:
   a determination of one or more schedule of the plurality of events associated with the clinical procedure. The schedule may comprise a linear sequence of events, the schedule may comprise a branching schedule of a sequence of events.
(iv) Evaluating, based on the clinical workflow information and case information,
   a projected elapsed time between the events of the one or more schedule of events associated with the clinical procedure. The projected elapsed time between events may comprise a predicted elapsed time between occurrence a first event and a second event of the plurality of events.
(v) Providing, based upon the evaluation, information relating to occurrence of an event of interest if provided with information relating to occurrence of another event associated with the clinical procedure. The information relating to occurrence of an event may comprise a relative or absolute time of occurrence of an event if provided with a time of occurrence of another event.

Embodiments in line with described approaches may support provision of a dedicated notification or alert system configurable to provide a user with, for example, a reduced amount of all available information. Some implementations of mechanisms and systems in accordance with approaches are such that an individual or entity can be notified a pre-defined time period, for example, a number of minutes, in advance of occurrence of an event of interest. Some embodiments may support an approach in which a user can select or "tag" an event, task or activity of interest which may occur as part of a clinical procedure. The selected or tagged event, task or activity, may be those which are of special interest in the clinical procedure.

An event of interest in a clinical procedure may vary in nature.

A non-exhaustive list of events which could be considered as an event of interest include, for example, in the context of an imaging procedure: start of patient/subject positioning; start of MRI image acquisition; a time point during, for example, MRI image acquisition when expertise for geometry planning is required (i.e. after survey scan); end of MRI image acquisition (when a remote expert could offer support or input by providing acquired image quality checks); when contrast agent is injected (which can be before or during, for example, an MRI exam); and/or when a specific complicated sequence is started during, for example, an MRI exam. Described embodiments may have particular utility in the context of an imaging or radiology workflow management method. That is to say, in relation to clinical procedures comprising imaging or radiology procedures.

A non-exhaustive list of events which could be considered as an event of interest include, for example, in a broader clinical procedure context: when informed consent from patient may need to be collected (thus allowing a person responsible for this step to be informed before the event occurs); the end of a process, for example, the end of an MRI exam (thus allowing a patient transport service to be informed of the event prior to it occurring); when a subject image or result is ready on a PACS (for example, to inform a clinical professional that their expert interpretation or input may be required); arrival of a subject or patient at a department, for example, a radiology department (for example, to inform staff to meet a patient); at a branch decision point in a clinical procedure, for example, when a decision is taken by a physician regarding a branch in the process (e.g., sedation or no sedation) (to allow such a physician to be alerted of their involvement in the branch decision action ahead of time).

Some implementations of mechanisms and systems in accordance with approaches may employ machine learning (ML) or artificial intelligence (Al) based event schedule prediction.

Some implementations of mechanisms and systems in accordance with approaches may employ machine learning (ML) or artificial intelligence (AI) based identification of one or more event associated with the clinical procedure which are likely to be of interest. Some implementations may provide for suggestion of one or more event(s) of interest in relation to the clinical procedure.

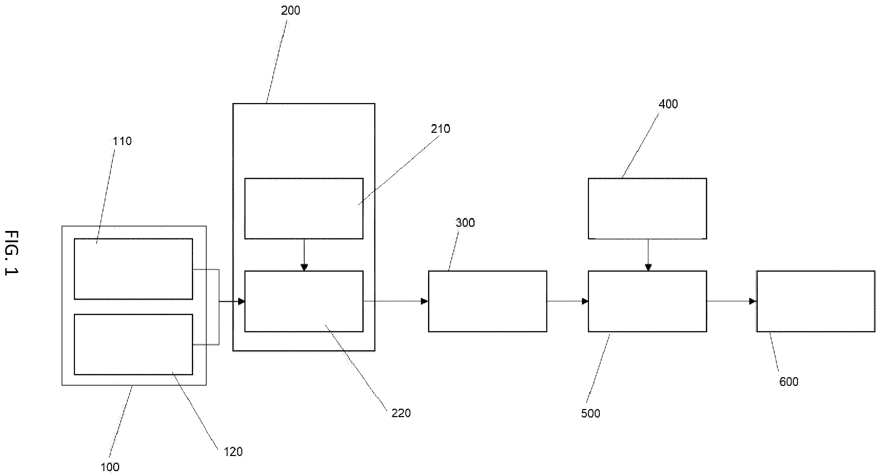

FIG. 1 illustrates schematically components of one embodiment of a system configurable to operate in accordance with some implementations of approaches described generally herein.

One or more event associated with a clinical procedure may occur. Information relating to an event may be known or generated by a system, apparatus or device involved in that event. Such systems and devices are shown generally in FIG. 1 as event information sources 100. Example information sources include, for example, devices and equipment 110 involved in a clinical procedure and/or clinical or healthcare information systems 120, such as a Radiology Information System (RIS), Picture Archiving and Communication System (PACS), Electronic Medical Record (EMR) and similar.

The devices and equipment 110 may be configured to capture real-time, or near real-time, information about events which form part of a process performed by the device or equipment. Devices can be imaging devices, such as an MRI scanner, x-ray machine, CT scanner or similar, providing information about a start and end of an examination or scanning process. Devices can be patient sample analysis devices, providing information about a sample receipt or arrival time, processing time, and start result availability time. Devices may include door card readers, providing information about patient entry or exit to a facility.

The clinical or healthcare information systems 120 may be configured to capture information including real time, or near real time, information relating to events which occur as part of clinical procedure. By way of example, a RIS may be configured to provide operational information, such as patient registration events or report generation events, an a PACS system may be configured to provide a notification when or if medical images generated as part of a clinical procedure are available for reading.

The event information sources 100 may be configured to provide information relating to events forming part of, or associated with, a clinical procedure to a workflow management system 200. The information provided to the workflow management system 200 may comprise clinical workflow information comprising a plurality of events associated with a clinical procedure and case information associated with a group of the plurality of events which together form an occurrence of the clinical procedure. The case information may comprise information relating to occurrence of at least some of the events in the group, for example, an indication of a relative or absolute time or time stamp associated with occurrence of an event.

The workflow management system 200 is configured to obtain or receive information relating to events from one or more event information sources 100. The workflow management system 200 may comprise an automated workflow management system.

The workflow management system may be configured to define clinical procedures, including all events, tasks, activities and resource assignments which may form part of such clinical procedures. In one embodiment, the workflow management system 200 may be configured to evaluate, based on provided event information, an indication of one or more schedule 210 of a plurality of events associated with the clinical procedure. That schedule 210 may serve to offer a definition or map of routes through events which together form a clinical procedure. The workflow management system may generate such a route map relating to a clinical procedure using a process modelling language, for example, BPMN 2.0.

The workflow management system may be configured to evaluate a time period between events of the one or more schedule of events associated with the clinical procedure. That evaluation may be based upon provided event information relating to the timing or occurrence of an event which forms part of the clinical procedure.

The workflow management system may be configured to track progress of an instance of a clinical procedure in, for example, real or near-real time, by mapping event information provided by event information sources 110, 120 to pre-defined clinical procedure schedules.

In other words, the workflow management system 200 may be configured to receive or generate, based on event information, a model of events which together form a clinical procedure. The model 210 may comprise map of routes through events which together form a clinical procedure. With knowledge of that model, the workflow management system may be configured to use event information provided to it, to monitor or track progress of a current or ongoing instance 220 of the clinical procedure made up of events. That is to say, the workflow management system 200 may be configured to provide information relating to occurrence of an event of interest if provided with information relating to occurrence of another event associated with the clinical procedure.

Some embodiments may extend the workflow management system 200 such that it includes a workflow prediction engine 300. Although shown as a separate block in FIG. 1, it will be appreciated that workflow prediction engine 300 may form part of the workflow management system 200.

The workflow prediction engine 300 may be configured to receive information relating to events which form part of a current instance of a clinical procedure. The event information provided to the workflow prediction engine 300 may comprise context information about a patient and events, for example, based on information provided by healthcare information systems such as an EMR or RIS.

Based on event information related to previous occurrences of a clinical procedure, the workflow prediction engine 300 may be configured to predict when or whether events in a schedule of events associated with the clinical procedure will take place and how long an event or events in the schedule of events might take. In other words, the workflow prediction engine may comprise a reasoning engine, and that reasoning engine may be configured to: obtain event information comprising clinical workflow information and case information and create a model or schedule of events representing the clinical procedure. The model and event information may together support evaluation of elapsed time between events which feature in the schedule.

It will be appreciated that the workflow prediction engine 300 may be configured to create the model of the clinical procedure using historic event information relating to a plurality of instances of a given clinical procedure. The event information may, as described previously, and depending upon implementation, comprise: clinical workflow information, case information, context information, key event information and similar.

The workflow prediction engine 300 may also be configured to analyse or assess event information provided relating to resource assignment for one or more event. That analysis or assessment may be used as an input in a prediction step performed by the workflow prediction engine 300. By way of example, if two instances of a clinical procedure require a "shared" resource (for example, if an event in an instance of a clinical procedure requires a room/equipment/staff member which may be concurrently required by another instance of a clinical procedure), a desire to avoid such collision of events, or to avoid concurrent resource need may be taken into account as a boundary condition in a prediction algorithm implemented by the workflow prediction engine 300.

A prediction algorithm implemented by the workflow management system 200 and/or, where provided, the workflow prediction engine 300, may take various forms. By way of example, two possible non-limiting implementations are described below:
A first possible implementation of a prediction algorithm can be based on prior knowledge available to the workflow management system. That is to say, a workflow management system 200 or prediction engine 300 may be provided with one or more schedule of events which are associated with a clinical procedure. An expected duration of events or groups of events may be determined by the workflow management system 200 or workflow prediction engine 300. That expected duration may be determined by appropriate application of a machine learning model, such as a neural network. That model may be trained on historic information relating to instances of the clinical procedure or events associated with a clinical procedure. In other words, the information used for training may comprise event information having features: patient/case context, process and activity type; and labels: event duration.

In this, and other, implementations of a prediction algorithm, an expected availability of resource may be determined or evaluated by combining an expected duration of an event or series of events and an evaluation of resource assignment obtainable from event information and the schedule of events defining the clinical procedure.

In an implementation where both event duration and resource availability are taken into account, the workflow management system 200 and/or workflow prediction engine may be configured to provide a prediction or estimation of start and end times of upcoming events in a clinical procedure.

A second possible implementation of a prediction algorithm can be based on self-learned schedule knowledge. According to such an implementation, a long-short-term memory (LSTM), other recurrent neural network (RNN), or similar machine learning algorithm provided as part of the workflow management system 200 or workflow prediction engine 300 can be configured to learn and predict a schedule of events in a clinical procedure and the event start and end times as, for example, a time series. In this implementation, no prior knowledge about the clinical procedure definition is required, because the machine learning model will infer this from historic time series of activities derived from event information provided.

Implementations may operate such that resource allocation associated with events may be taken into account for the prediction of timing of other events in a schedule. For each event or set of events which form part of a clinical procedure model, the required resource(s) for performance of that event may be known. For example, if two events or procedures (set of events) require the same resource, such as a specific room or piece of equipment, one event may be delayed while the resource is used by the other event. Including resource allocation in the event information provided to the workflow management system or workflow prediction engine may allow for more accurate time prediction of, or between, events forming part of a schedule of events which together form an occurrence of a clinical procedure.

According to some prediction algorithm implementations, a system may be configured to provide various types of additional information in relation to an event of interest. Two possible types of additional information comprise a determination of confidence in a time prediction associated with occurrence of an event of interest and a determination of probability of a time prediction associated with occurrence of an event of interest as set out in more detail below. Confidence in a prediction may refer to how exact the prediction of the expected time for an event is. According to some implementations, the confidence determination could be specified as a 95% confidence interval in minutes around a central predicted time. If the underlying schedule which defines a clinical procedure has branches and the event of interest can be reached via different paths or sequences of events in that schedule, the confidence can either reflect the total range of expected times for all possible paths, or could be specified for each path separately. The latter may be beneficial if there is one path with a very high branching probability (see below). One way of determining the confidence interval may be basing the confidence interval upon statistics associated with historic similar cases. For all upcoming workflow steps until an event of interest in a given schedule, a distribution of durations of historic similar cases can be determined. The width of the 95 percentile of such a determined distribution can be used to estimate a 95% confidence interval.

Probability of a prediction may refer to a probability of an event in a schedule occurring at all. The probability of an event occurring may depends on the probabilities of any schedule branches before reaching an event of interest and the probability for the schedule to terminate and end prematurely. For a linear schedule of events without branches, the probability of an event of interest to occur may depend only upon the probability that the schedule (clinical procedure) itself does or does not end prematurely. A probability of an event of interest which occurs later in a schedule could be considered as a product of all branching probabilities from a currently active event to the event of interest. For each branch in the schedule on the way between a current event and an event of interest, branching probabilities can be inferred from historical cases based on knowledge about the instance of occurrence of the clinical procedure up to the current event. If two or more paths through a complex schedule lead to the event of interest, their individual probabilities need to be added. Implementations in which a determination of probability associated with a time prediction of an event of interest is provided may contain a factor or indication which specifies a likelihood of (non-)termination of the schedule.

In some implementations, information associated with a time prediction of an event of interest, such as prediction confidence and prediction probability, may be provided to a system user. If multiple paths lead to the event of interest, confidence and probability can be given either as total values (summing over all paths) or for each path separately. In some implementations, such information can be updated when a current occurrence of a clinical procedure proceeds from an event in a schedule to another event in the schedule, and/or if context information associated with an event changes.

The system of FIG. 1 includes an event tagging interface 400 and alert generator 500. The event tagging interface 400 may be configured to receive an indication, for example, from a healthcare professional or other user of the clinical workflow management system, of one or more event of interest in an ongoing occurrence of a clinical procedure. The system may then use information provided by the management system 200 or prediction engine 300 to provide a user with information relating to occurrence of the one or more event of interest based on provision of information relating to occurrence of another event in the ongoing occurrence of the clinical procedure. In particular, the alert generator 500 may be configured to receive input from the event tagging interface and management system or prediction engine to generate an alert relating to a "tagged" event of interest. The alert generator 500 may communicate, for example, with a notification device 600 which is, in turn, operable to provide a user with information relating to occurrence of the event of interest.

According to some implementations of approaches described herein, a person interested in being notified of occurrence of an event during a clinical procedure associated with a specific patient can interact with the workflow management system and prediction engine via the event tagging interface 400. The event tagging interface 400 allows a system user to enter the details of an event notification request via a user interface. The information a user may provide in support of an event notification request may comprise, but is not limited to: a patient reference; and event in the clinical procedure which is of interest (event of interest which is to be "tagged"); a reference time (for example, a start or end of an event of interest); and a notification time relative to reference time (for example, a number of minutes before or after the event of interest).

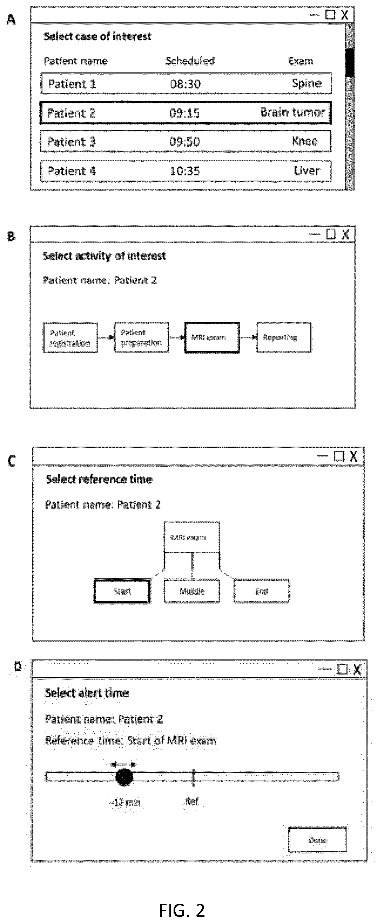

FIG. 2 is a representation of a possible user interface according to one implementation which may be displayed to a user by the event tagging interface 400 shown in FIG. 1.
(A) In a first step, a user can select a patient from a list of scheduled patients. It can be seen that the selection may comprise context information associated with the patient. In this example, information may include a type of examination required in relation to the patient and an indication of a scheduled event associated with that patient.
(B) In a second step, a user may interact with the event tagging interface to select ("tag") an event of interest from the clinical procedure model (schedule of events) associated with the selected patient. In this example, the user selects the MRI examination as being an event of interest.
(C) In a third step, the user may select a reference time in relation to the tagged event, for example, the start, middle, or end of the selected event.
(D) In a fourth step, the user may input a desired alert time relative to the selected reference time. In the example shown here, the alert time is set to 12 minutes prior to the reference time (indicated by a negative number). The alert time can also be later than the reference time (indicated by a positive number).

In addition to the information provided in the example user interface shown in FIG. 2, a user may, in some implementations of the event tagging interface, be able to specify a preferred means of notification, for example, a push message to phone, an email, an on-screen notification or similar.

In some implementations, the event tagging interface may be configured to generate an alert based upon the information relating to occurrence of the event of interest. In some implementations, the event tagging interface may be configured to notify a user of a projected occurrence of the event of interest. In some implementations, the event tagging interface may be configured to notify the user a predetermined period before likely occurrence of the event of interest. In some implementations, the event tagging interface may be configured to communicate with the notification device(s) 600 such that the device(s) generate a visual, audio or haptic notification or alert.

Returning to the System schematic of FIG. 1, in some implementations, the alert generator 500 may be configured to compare a notification request(s) provided by a user to the event tagging interface 400 with an actual time and a predicted time of an event of interest. If a negative alert time has been chosen by a user (a time before the reference time of an event of interest), the alert generator 500 may be configured to use a predicted time of an event of interest to determine whether the alert condition is met. If a positive alert time is chosen by a user (a time after the reference time of an event of interest), the alert generator may be configured to use an actual time associated with the event of interest to determine whether an alert condition is met.

If the alert generator 500 determines that an alert condition is met (the current time matches a requested alert time in relation to an event of interest), the alert generator may be configured to provide appropriate signalling to a notification device 600. In this way, a notification of an event of interest can be communicated to a user. Notifications can be implemented in different ways, including, but not limited to: push messages to mobile devices; a message in a messenger app on a mobile device; email; on-screen notification provided by an operating system; on-screen notification provided by a web interface; an acoustic signal from a mobile device or loudspeaker in a room or location within a healthcare facility; an optical signal (for example, a flashing light); and/or a haptic signal (for example, vibration of a mobile device).

In some implementations, it will be understood that notifying a user comprises transmitting a notification to a remote user. Some implementations may provide that a system such as that shown schematically in FIG. 1 may be configured to receive input from a user in response to notification of information relating to occurrence of the event of interest. The input from the user may, for example, comprise an acknowledgement of the notification and/or information relating to one or more event of the plurality of events associated with the clinical procedure. By way of example, a clinician may be able to indicate their availability or otherwise in relation to an event associated with the clinical procedure. Provision of such information may allow the system to update prediction of time in relation to an event in the clinical procedure.

In some implementations, the event tagging interface 400 may be provided with additional functionality. In particular, provision may be made such that the event tagging interface may obtaining key event information associated with a plurality of occurrences of the clinical procedure, the key event information comprising a determination one or more event of interest to a user. The event tagging interface may be configured to provide a user with a suggestion of one or more event of interest in a clinical procedure based upon the obtained key event information. Some implementations involve provision of an appropriate reasoning engine and configuring the reasoning engine to: obtain the key event information and clinical workflow information; and generate a model of probable events of interest to a user of the plurality of events associated with the clinical procedure. The model of the clinical procedure is generated using historic clinical workflow information and key event information.

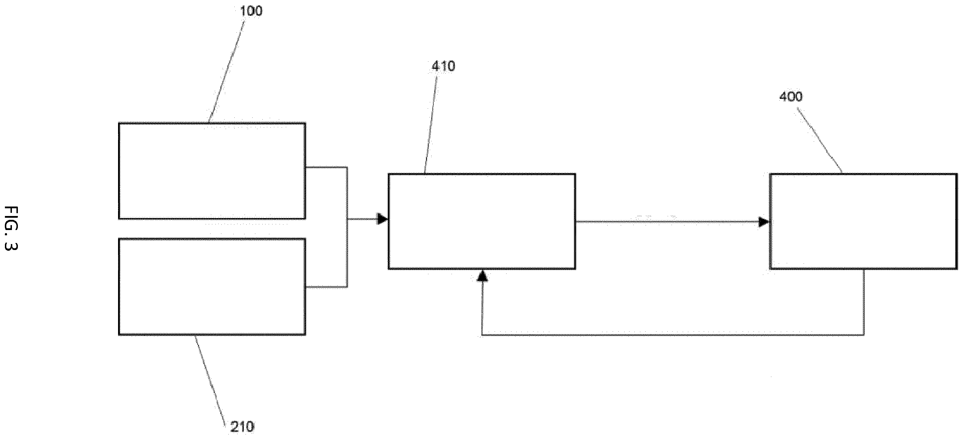

FIG. 3 illustrates schematically one possible mechanism for providing an event tagging interface with functionality such as that described above. Such an implementation may mean that notification request parameters such as those described in relation to FIG. 2 can be proposed or pre-populated by a machine learning algorithm based on prior notification requests.

In such an implementation, a user may not need to go through all steps of specifying a notification request manually, but instead (or in addition) could be provided with one or several proposed notification request definitions by the event tagging interface 400.

For example, a radiologist frequently asking to be notified when a specific type of spine examination has been completed is likely, in such an implementation, to have the event tagging interface propose corresponding event tagging and notification if a similar clinical procedure is scheduled.

One possible implementation of an AI-based tagging proposal system is shown schematically in FIG 3. As shown in FIG. 3, an AI engine 410 may be configured to communicate with the event tagging interface 400 and with the workflow management system 200 to receive clinical procedure models 210 and event information 100. The AI engine 410 may be configured to use event information, for example, patient and case context information from a RIS, EMR, or similar hospital IT system. The AI engine 410 may also be configured to receive or use clinical procedure schedules 210 known to, or determined by, the workflow management system 200. The AI engine may also receive event tagging details (key events which have been tagged as events of interest in previous instances of use of the event tagging interface 400). Based on the provided information and training data from the event tagging interface, the AI engine 410 may model the events in a given schedule associated with a clinical procedure which are likely to be tagged by a user as being an event of interest. As a result, the AI engine may be operable to propose tagging details to the event tagging interface 400. Those tagging details may comprise which, if any, event in a schedule to tag and which reference and alert times to choose in relation to that event. The proposed parameters can be pre-populated in the user interface presented above in relation to FIG. 2 or can be displayed to a user in a dedicated user interface. Implementations may allow a user to deviate from proposed tagging and alert/notification parameters associated with an event. The AI engine 410 may be configured to use actual tagging details, for example, actual tagging details may be provided by event tagging interface 400, (reviewed and possibly adapted by a user) as training input for the Al algorithm. In this way, the tagging prediction algorithm may steadily improve its proposals to a user.

A system such as that described above may facilitate the following uses: a radiologist may be informed 10 minutes prior to the start of an MRI examination of a specific patient, thus allowing the radiologist to align protocol details with a technologist performing the examination; a radiologist may be informed 15 minutes prior to the end of an MRI examination of a specific patient, to ensure they are available to read the captured images promptly; a technologist or nurse may be informed 5 minutes before an expected arrival of a patient at a radiology department's reception desk so that the patient can be met directly upon arrival at the department.

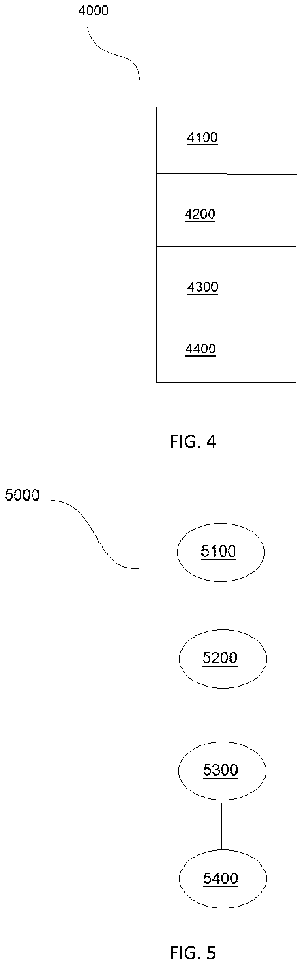

FIG. 4 is a schematic representation of an apparatus according to one implementation; and FIG. 5 is a schematic representation of a method according to one implementation.

FIG. 4 shows an apparatus 4000 comprising: circuitry 4100 configured to obtain clinical workflow information comprising a plurality of events associated with a clinical procedure; circuitry 4200 configured to obtain case information associated with a group of the plurality of events which together form an occurrence of the clinical procedure, the case information comprising information relating to occurrence of at least some of the events in the group; circuitry 4300 configured to evaluate, based on the clinical workflow information and case information: a determination of one or more schedule of the plurality of events associated with the clinical procedure; and a time difference between the events of the one or more schedule of events associated with the clinical procedure; and circuitry 4400 configured to provide, based upon the evaluation, information relating to occurrence of an event of interest if provided with information relating to occurrence of another event associated with the clinical procedure.

FIG. 5 is a schematic representation of a method according to one implementation. According to the method 5000 shown in FIG. 5, a method according to an implementation may comprise steps of:
5100: obtaining clinical workflow information comprising a plurality of events associated with a clinical procedure;
5200: obtaining case information associated with a group of the plurality of events which together form an occurrence of the clinical procedure, the case information comprising information relating to occurrence of at least some of the events in the group;
5300: evaluating, based on the clinical workflow information and case information: a determination of one or more schedule of the plurality of events associated with the clinical procedure; and a time difference between the events of the one or more schedule of events associated with the clinical procedure; and
5400: providing, based upon the evaluation, information relating to occurrence of an event of interest if provided with information relating to occurrence of another event associated with the clinical procedure.

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods. The term non-transitory as used herein, is a limitation of the medium itself (i.e., tangible, not a signal) as opposed to a limitation on data storage persistency (e.g. RAM vs ROM).

As used in this application, the term "circuitry" may refer to one or more or all of the following:
(a) hardware-only circuit implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and or processor(s), such as a microprocessor(s) or a portion of a microprocessor(s), that requires software (e.g., firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in server, a cellular network device, or other computing or network device.

While the invention has been illustrated and described in detail in the drawings and foregoing description, such illustration and description are to be considered illustrative or exemplary and not restrictive; the invention is not limited to the disclosed embodiments.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method (5000) for clinical workflow management, the method comprising:
obtaining (5100) clinical workflow information comprising a plurality of events associated with a clinical procedure;
obtaining (5200) case information associated with a group of the plurality of events forming an occurrence of the clinical procedure, the case information comprising information relating to occurrence of at least some of the events in the group of the plurality of events;
evaluating (5300), based on the clinical workflow information and case information:
an indication of one or more schedule of the plurality of events associated with the clinical procedure; and
a time difference between the events of the one or more schedule of events associated with the clinical procedure; and
providing (5400), based upon the evaluation (5300), information relating to occurrence of an event of interest if provided with information relating to occurrence of another event associated with the clinical procedure.

2. A method according to claim 1, comprising:
providing a reasoning engine and configuring the reasoning engine to:
obtain the clinical workflow information and case information; and
create a model of the clinical procedure by evaluating the indication of one or more schedule and time difference between events.

3. A method according to any one of claim 1 or claim 2, wherein the case information comprises resource scheduling information relating to occurrence of at least some of the events, and wherein the evaluated time difference between events is further based upon the resource scheduling information.

4. A method according to any one of the preceding claims, wherein the information relating to occurrence of another event associated with the clinical procedure comprises: real-time or near real-time information relating to an ongoing occurrence of the clinical procedure.

5. A method according to any one of the preceding claims, wherein the method comprises:
monitoring a clinical procedure based upon the provided information relating to occurrence of an event of interest.

6. A method according to any one of the preceding claims, wherein providing information relating to occurrence of the event of interest comprises a determination of whether the event of interest will occur.

7. A method according to any one of the preceding claims, wherein providing information relating to occurrence of the event of interest comprises providing a determination of confidence or probability associated with the provided information.

8. A method according to any one of the preceding claims, wherein the method comprises:
receiving a determination of one or more event of interest in an ongoing occurrence of the clinical procedure; and
wherein providing the information relating to occurrence of the one or more event of interest comprises providing the information in response to provision of information relating to occurrence of another event in the ongoing occurrence of the clinical procedure.

9. A method according to any one of preceding claims, wherein the method comprises:
notifying a user of the information relating to occurrence of the event of interest.

10. A method according to claim 9, wherein the method comprises:
receiving an input from a user in response to notification of information relating to occurrence of the event of interest, the input from the user comprising information relating to one or more events of the plurality of events associated with the clinical procedure.

11. A method according to any one of the preceding claims, comprising:
obtaining key event information associated with a plurality of occurrences of the clinical procedure, the key event information comprising a determination of one or more event of interest to a user; and
providing a user with a suggestion of one or more event of interest in a clinical procedure based upon the obtained key event information.

12. A method according to claim 11, wherein the method comprises: providing a reasoning engine and configuring the reasoning engine to:
obtain the key event information and clinical workflow information; and
generate a model of probable events of interest to a user of the plurality of events associated with the clinical procedure.

13. A method according to claim 12, wherein the model of the clinical procedure is generated using historic clinical workflow information and key event information.

14. A non-transitory computer readable medium comprising program instructions stored thereon for performing at least the following:
obtaining clinical workflow information comprising a plurality of events associated with a clinical procedure;
obtaining case information associated with a group of the plurality of events forming an occurrence of the clinical procedure, the case information comprising information relating to occurrence of at least some of the events in the group of the plurality of events;
evaluating, based on the clinical workflow information and case information:
an indication of one or more schedule of the plurality of events associated with the clinical procedure; and
a time difference between the events of the one or more schedule of events associated with the clinical procedure; and
providing, based upon the evaluation, information relating to occurrence of an event of interest if provided with information relating to occurrence of another event associated with the clinical procedure.

15. An apparatus (4000) comprising:
at least one processor; and
at least one memory storing instructions that when executed by the at least one processor cause the apparatus at least to perform:
obtaining clinical workflow information comprising a plurality of events associated with a clinical procedure;
obtaining case information associated with a group of the plurality of events forming an occurrence of the clinical procedure, the case information comprising information relating to occurrence of at least some of the events in the group of the plurality of events;
evaluating, based on the clinical workflow information and case information: an indication of one or more schedule of the plurality of events associated with the clinical procedure; and
a time difference between the events of the one or more schedule of events associated with the clinical procedure; and
providing, based upon the evaluation, information relating to occurrence of an event of interest if provided with information relating to occurrence of another event associated with the clinical procedure.
